Europäisches Patentamt

European Patent Office

Office européen des brevets

(11) Veröffentlichungsnummer : **0 356 900 B1**

(12) **EUROPÄISCHE PATENTSCHRIFT**

(45) Veröffentlichungstag der Patentschrift :
**25.11.92 Patentblatt 92/48**

(51) Int. Cl.⁵ : **A61K 37/66**

(21) Anmeldenummer : **89115547.5**

(22) Anmeldetag : **23.08.89**

(54) **Verwendung von IFN-gamma enthaltenden Arzneimitteln zur Vorbeugung und Behandlung von durch primäre Immundefekte verursachte Krankheiten.**

(30) Priorität : **29.08.88 DE 3829180**

(43) Veröffentlichungstag der Anmeldung :
**07.03.90 Patentblatt 90/10**

(45) Bekanntmachung des Hinweises auf die
Patenterteilung :
**25.11.92 Patentblatt 92/48**

(84) Benannte Vertragsstaaten :
**AT BE CH DE ES FR GB GR IT LI LU NL SE**

(56) Entgegenhaltungen :
**EP-A- 0 170 917**
**EP-A- 0 219 781**
**EP-A- 0 254 593**

(56) Entgegenhaltungen :
**THE NEW ENGLAND JOURNAL OF MEDICINE
vol. 318, no. 20, 19 Mai 1988, Massachusetts
Seiten 1295 - 1300; DE PREVAL C. et
al:"Regulation of genes for HLA class II antigens in cell lines from patients with severe
combined immunodeficiency"
CLINICAL AND EXPERIMENTAL IMMUNO-
LOGY vol. 72, no. 1, 1988, OXFORD Seiten 124 -
129; PAGANELLI R. et al: "Evidence thatdefective gamma interferon production in patients with primary immunodeficiencies is due
to intrinsic incompetence of lymphocytes."
BIOLOGICAL ABSTRACT Nr: 81082268
STRASSMANN G. et al:"Biochemical models
of interferon gamma-mediated macrophage
activationindependent regulation of lymphocyte function associated antigen 1 and I-A
antigen on murine peritoneal macrophages."
&CELL.IMMUNOL. 97 (1) 1986, p 110-120**

(73) Patentinhaber : **BOEHRINGER INGELHEIM
INTERNATIONAL G.M.B.H.
W-6507 Ingelheim am Rhein (DE)**

(72) Erfinder : **Fasth, Anders, Dr. Department of
Pediatrics
University of Göteborg East Hospital
S-41685 Göteborg (SE)**

EP 0 356 900 B1

## Beschreibung

Die Erfindung bezieht sich auf ein Interferon gamma (IFNγ) enthaltendes Arzneimittel, zur Verhütung und Behandlung von Krankheiten, die durch primäre Immundefekte verursacht werden. Insbesondere richtet sich das Arzneimittel gegen Krankheiten, die aufgrund einer "schweren kombinierten Immundefizienz (SCID)" regelmäßig oder sporadisch auftreten können.

Primäre Immundefekte bedingen ein stark erhöhtes Infektionsrisiko. Bei den schwersten Formen können die Infektionen schon während des ersten Lebensjahres zum Tod des betroffenen Patienten führen.

Unter primären Immundefekten werden hier solche Immundefekte verstanden, die genetisch determiniert sind und vererbbar sein können und von den sekundären Immundefekten zu unterscheiden sind, die im Zusammenhang mit anderen Ereignissen (z.B. Virusinfektionen, Medikamentenbehandlungen) als Folgeerscheinung manifest werden.

Die hier angesprochenen primären Immundefekte umfassen sowohl die humoralen und zellulären, als auch die kombinierten Immundefekte, die durch Funktionsausfälle bei der humoralen und zellulären Immunantwort gekennzeichnet sind. Dazu gehören auch jene angeborenen Krankheiten, die zu einer verminderten Reifung der funktionellen, immunkompetenten Zellen oder zu einer verminderten Expression der für die Interaktion erforderlichen Antigene auf der Zelloberfläche führen (z.B. LFA 1 Defizienz Syndrom).

Typische immunologische Befunde bei humoralen Immundefekten sind u.a. das vollständige Fehlen von sowohl zirkulierenden B-Lymphozyten als auch von B-Lymphozyten und Plasmazellen in den Lymphknoten, der Milz und anderen lymphatischen Systemen. Der klassische primäre zelluläre Immundefekt zeigt demgegenüber ein partielles oder vollständiges Fehlen von T-Lymphozyten im peripheren Blut mit einhergehender schwerer Lymphopenie, eine verringerte Anzahl an Lymphozyten in den entsprechenden Lymphorganen (z.B. Milz, Lymphknoten) und eine verminderte Fähigkeit, zelluläre Immunantworten durchzuführen.

Neben diesen mehr oder weniger isolierten Immundefekten, die schon für sich alleine zu einem beträchtlich erhöhten Infektionsrisiko führen und sowohl extrazelluläre als auch intrazelluläre Erreger wie Viren, Pilze, Bakterien (Mykobakterien) umfassen, führen die primären kombinierten Immundefekte zu einem Verlust der Fähigkeit, humorale und zelluläre Immunantworten durchzuführen - mit praktisch immer letalen Folgen für die betreffenden Patienten.

Fatal für diese Patienten wirkt sich aus, daß die Immunisierung mit verschiedenen Antigenen, die bei normalem Immunstatus eine Antikörperbildung induzieren, zu keinerlei immunologischen Antwort führt. Die primären kombinierten Immundefekte manifestieren sich in einem Krankheitsbild, das durch rezidivierende und schwerste Infektionen durch Viren, pilze und Bakterien beherrscht wird. Die Pilzinfektionen werden z.B. durch Candida und Pneumocystis Arten verursacht, die Pneumonien hervorrufen können. Die Empfänglichkeit für Opportunisten-Infektionen ist hoch. Weiterhin treten häufig Dystrophien und Symptome im gastrointestinalen Trakt mit schweren Diarrhoen auf. Der sehr frühe Beginn dieser Infektionen ist charakteristisch, und die Patienten zeigen im Verlauf der neonatalen Phase keine normale Entwicklung bzw. Gewichtszunahme. Darüberhinaus liegt eine Störung der Phagozytosefähigkeit von Granulozyten bzw. Monozyten vor. Die Prognose für die Patienten ist sehr schlecht bis infaust, da die Infektionen bisher nicht oder nur sehr unzureichend beherrscht werden konnten. Viele Patienten versterben daher frühzeitig an den Folgen dieser multifaktoriellen, polytopen Infektionen.

Als pathognomonische Befunde fallen histologisch bei diesen Patienten ein mehr oder weniger hypoplastisches lymphoides Gewebe und ein mehr oder weniger embryonaler Thymus auf. Sowohl B- als auch T-Zellareale des peripheren Lymphgewebes sind hinsichtlich der Lymphozyten stark dezimiert.

Einer der Hauptgründe dafür, daß bisher keine wirkungsvollen Therapien dieser Infektionskrankheiten existieren, insbesondere bei SCID, ist das praktisch vollständigen Fehlen aller immunologischen Funktionen.

Die bis heute bekannten therapeutischen Maßnahmen sind insbesondere auf eine Kompensation des Immundefektes ausgerichtet. Bekannt wurden z.B. Knochenmarktransplantationen, mit der Zielvorstellung, daß sich die Stammzellen im Empfänger zu reifen T- und B-Zellen entwickeln sollen. Dabei handelt es sich jedoch um eine sehr risikoreiche Maßnahme, da mit schweren "Graft-Versus-Host" Reaktionen zu rechnen ist, wenn Spender und Empfänger nicht genau kompatibel sind. Demzufolge ergibt sich das große Problem, einen geeigneten Spender zu finden, bevor es zu einer letal verlaufenden Infektion kommt. Therapeutische Maßnahmen zur Bekämpfung der rekurrierenden und persistierenden Infektionen stehen bis heute nicht zur Verfügung, da z.B. selbst eine antibiotische Behandlung ohne die gewünschte Wirkung bleibt.

Es soll nochmals hervorgehoben werden, daß sich der hier angesprochene Krankheitskomplex klar von solchen Erkrankungen abgrenzt, die durch z.B. von Retroviren verursachte Folgeinfektionen oder durch gewöhnliche Infektionen bei sonst normalem Immunstatus hervorgerufen werden und durch herkömmliche, bekannte Methoden einer Behandlung zugänglich sind.

Die zu lösende Aufgabe der vorliegenden Erfindung bestand darin, ein Mittel zu finden, welches eine wirksame Therapie der durch primäre Immundefekte, insbesondere durch primäre kombinierte Immundefekte hervorgerufene Infektionen ermöglicht, welches ein Ausbrechen solcher Infektionen verhindert oder welches durch Kompensation dieser genetisch bedingten Immundefekte erst die Voraussetzungen für die Wirksamkeit begleitender z.B. antibiotischer Maßnahmen schafft.

Völlig überraschend wurde gefunden, daß durch die erfindungsgemäße Verwendung von Interferon gamma (IFNγ) die vorbeschriebenen, schweren Komplikationen verhütet, beherrscht und zur Heilung gebracht werden können. Es zeigte sich, daß mit IFN γ die fortschreitende Infektion zum Stillstand gebracht wird und eine Abheilung der durch sie verursachten Läsionen erzielt werden konnte.

Die Erfindung umfaßt demzufolge die Vorbeugung und Behandlung von Komplikationen, verursacht von angeborenen Störungen, die zu einer verminderten Reifung der funktionellen, immunkompetenten Zellen oder zu einer verminderten Expression der für die Interaktion erforderlichen Antigene auf der Zelloberfläche führen, wie beispielsweise beim LFA 1 Defizienz Syndrom (Leucocyte Function Antigen) der Fall ist.

Diese durchgreifende Wirkung war aufgrund der vom Stand der Technik bekannten Eigenschaften des Immunodulators IFN γ nicht zu erwarten. Denn IFN γ selbst übt keine direkte Wirkung auf Erreger wie z.B. Viren aus. Vielmehr wirkt IFN γ als biologischer "messenger", dessen Funktion eher darin zu liegen scheint, funktionstüchtige Bestandteile des bestehenden Immunsystems zu aktivieren oder zu stimulieren. Schon bei immunsupprimierten Patienten wird die Reaktivität als unzureichend betrachtet (Borecky, L. Fuchsberger, N., Acta Virol. 27, 359-370, 1983).

Die Publikation, von Paganelli, R. et al., Clin. exp. Immunol. 72, 124-129, 1988, weist aufgrund von in vitro, Untersuchungen auf eine Korrelation zwischen primären Immundefekten und der von T-Lymphozyten gesteuerten mangelhaften Produktion von Interleukin und Interferon hin. Es wird ferner ausgeführt, daß primäre Immundefekte auf eine unterdrückte Funktion von T-Lymphozyten zurückgeführt werden, welche wiederum die Produktion von Lymphokinen (wie etwa des γ-Interferons) bei anderen Zelltypen regulieren.

Von Strassmann G. et al., Cell. Immunol. 97 (1), 110-120, 1986, wird berichtet, daß IFN-γ die Expression von Zelloberflächenproteinen (HLA Klasse II und LFA-1 Antigene) modulieren bzw. induzieren kann, wobei postuliert wird, daß IFN-γ in Makrophagen molekulare Ereignisse ungeklärter Natur auszulösen vermag.

In der Publikation von De Préval, C. et al., The New England Journal of Medicine 318, 1295-1300, 1988, wird über die Wirkungslosigkeit von IFN-γ bei in vitro Tests zur Aktivierung von HLA Klasse II Antigenen berichtet und hervorgehoben, daß IFN-γ bei Patienten mit SCID wirkungslos sein wird.

Bei Patienten mit dem geschilderten schweren Krankheitsbild eines SCID, bei denen praktisch kein funktionierendes Immunsystem vorhanden ist, war aus genannten Gründen erst recht zu erwarten, daß IFN γ keine Wirkung zeigt und eine IFN γ-Behandlung eher als aussichtslos einzuschätzen wäre.

Der bisher bekannte Einsatzbereich von IFN γ umfaßt einen Patientenkreis mit Tumorerkrankungen und Infektionskrankheiten, mit normalem Immunstatus oder mit Immunkrankheiten, die völlig verschieden sind von dem Krankheitskomplex, welcher der hier vorliegenden Erfindung zugrundeliegt.

In der EP-A 254 593 wird vorgeschlagen, IFNγ bei sogenannten "HUIFN-gamma susceptive diseases" einzusetzen - also bei Krankheiten, bei denen von vornherein feststeht, daß sie durch IFN γ verhinderbar oder behandelbar sind. Neben den herkömmlichen viralen Erkrankungen und Tumorerkrankungen, werden auch Immunopathien aufgeführt, worunter der Fachmann Autoaggressionskrankheiten bzw. Immunkomplexkrankheiten versteht. Dementsprechend werden in der EP-A 254 593 atopische Allergie, Myoasthenia, Kollagenose, perniziöse Anämie, artikulärer Rheumatismus und systemischer Lupus erythematosus aufgezählt. Es wird aber weder die Lehre gegeben noch wird es dem Fachmann nahegelegt, IFNγ bei Erkrankungen einzusetzen, bei denen eine IFNγ-Therapie aussichtslos erscheinen muß, wie es für die Begleiterkrankungen bei primären Immundefekten, insbesondere bei SCID, begründeterweise bisher der Fall zu sein schien.

Für die Realisierung der vorliegenden Erfindung stehen heute dem Fachmann eine Vielzahl an Möglichkeiten zur Verfügung, IFN γ sowohl über konventionelle Methoden als auch über DNA-Rekombinationen herzustellen. Wählt er den erstgenannten Weg, kann er sich beispielsweise der Methode nach Yip. Y.K. Infect. Immun. 34, 131-39, 1981 bedienen. Für das in neuerer Zeit attraktivere Verfahren, authentisches human IFN γ über DNA-Rekombination entweder in prokaryotischen oder in eukaryotischen Systemen herzustellen, kann z.B. nach Gray P.W. et al. Nature 295, 503-508, 1982; Derynck, R. et al. Nucl. Acid Res. 11, 1819-37, 1983; Simons, G. et al. Gene 28, 55-64, 1984; Scatill, J. et al. Proc. Nat. Acad. Sci. USA 80, 4654-58, 1983; oder nach EP-A 77 670, EP-A 254 345, EP-A 273 373 verfahren werden. Die Erfindung schließt daher auch jedwede IFN γ Derivate ein, die nach an sich bekannten Methoden herstellbar sind (z.B. EP-A 170 917, EP-A 219 781). Ebenfalls eingeschlossen sind IFNγ Polypeptide, die über bekannte synthetische Verfahren auf der Protein- und DNA-Ebene herstellbar sind (z.B. EP-A 161 504; Tanaka S. et al. Nucl. Acids Res. 11, 29-32, 1982)

Bevorzugt sind IFN γ-Präparate, die über Methoden der DNA-Rekombination gewonnen werden, da diese Verfahren in der Regel zu qualitativ und quantitativ besseren Ausbeuten führen.

Sowohl natürliches, synthetisches, semisynthetisches als auch rekombinantes IFN γ bzw. daraus herge-

3

stellte pharmazeutische Mittel können entweder systemisch (z.B. i.v., i.a., i.m.) und/oder, wenn es sich um sehr lokale Infektionsherde handelt, topisch in oder an den Infektionsort verabreicht werden.

Die Dosierung richtet sich dabei nach dem Schweregrad der Erkrankung bzw. nach dem Gesundheitszustand des Patienten.

Da es sich bei der betreffenden Patientengruppe aber in der Regel um Neugeborene oder Kleinkinder mit abnormem Entwicklungsstatus, weniger um Erwachsene, handeln wird, die erfindungsgemäß zu therapieren sein werden, richtet sich die Dosis vorzugsweise entweder nach der Körperoberfläche (wenn >0,5 m$^2$) oder nach dem Körpergewicht (wenn die Körperoberfläche <0,5m$^2$). Im allgemeinen kann für den ersten Fall davon ausgegangen werden, daß Injektionen oder Infusionen von IFN $\gamma$ einer spezifischen Aktivität von 1 bis 2 x 10$^7$ I.U./mg im Dosisbereich von 0,01 mg/m$^2$ bis 2 mg/m$^2$, entsprechend ca. 2 x 10$^5$ I.U. bis ca. 4 x 10$^7$ I.U./m$^2$, vorzugsweise von 0,05 bis 0,5 mg/m$^2$, insbesondere von 0,1 bis 0,2 mg/m$^2$, wirksam sind. Für den zweiten Fall ist ein Einzeldosisbereich von 0,15 µg/kg bis 0,015 mg/kg zu nennen, vorzugsweise von 0,5 µg/kg bis 5,0 µg/kg insbesondere von 1,5 µg/kg. Beispielsweise erfolgt die parenterale Anwendung 3 x wöchentlich. Abhängig vom klinischen und immunologischen Verlauf der jeweiligen Erkrankung kann die Behandlungsdauer bis zu 12 Monate oder länger betragen.

Um die Bekämpfung bzw. Ausheilung der Infektionen zu unterstützen, kann es zweckmäßig sein, die IFN$\gamma$-Therapie mit dem Fachmann bekannten antibiotischen oder chemotherapeutischen antibakteriellen, antiviralen oder antimykotischen Maßnahmen zu kombinieren. Sowohl eine konsekutive, eine alternierende als auch eine gleichzeitige parenterale oder enterale Gabe von mindestens einem bakteriostatisch oder bakteriozid wirkenden Antibiotikum und/oder eines Virostatikums und/oder eines Antimykotikums in Kombination mit IFN$\gamma$ (z.B. Sulfanilamide, Tetrazykline, Makrolide, Penizilline und Cephalosporine, Aminoglykoside, Rifamycine, Bacitracin und Polymyxine bzw. Aciclovir, Purinderivate bzw. Griseofulvin, Amphotericin, Clotrimazol, um nur einige zu nennen), auch in Kombination miteinander, wird erfindungsgemäß miteingeschlossen. Auch eine präventive Maßnahme kann auf dieser Grundlage zweckmäßig sein, um das Ausbrechen einer Erstinfektion oder einer zusätzlichen, weiteren Infektion zu verhindern.

Zur Herstellung der jeweiligen Darreichungsform, insbesondere Lösungen für die Injektion oder Infusion von IFN$\gamma$, werden die dem Fachmann bekannten Hilfsstoffe verwendet.

Das folgende Beispiel soll die Verwendung und Wirksamkeit von IFN $\gamma$ bei einem besonders schwerwiegenden Fall zeigen, den Gegenstand der Erfindung aber in keiner Weise einschränken.

Patient:
8 Monate altes Mädchen (Geburtsgewicht 1120 Gramm) mit erniedrigten T-Zellen, keiner mitogenen oder allogenen Immunantwort, hoher Anzahl an B-Zellen, mit Aberration an Chromosom 1 im heterochromatischen Bereich, und mit deutlich mentaler Retardierung.

Diagnose:
SCID, festgestellt am Tage der Geburt.

Komplikation:
Entwicklung einer schweren Ulzeration des Zungenbodens während des siebten Lebensmonats aufgrund einer viralen Infektion, chronischer Ausscheider von Echoviren in den Faeces.

vorhergehende
Therapie: Substitution mit Immunoglobulin, prophylaktische Gabe von Bactrim®(Thrimethoprim plus Sulfamethoxazol), dennoch weiteres Fortschreiten der Läsion.

IFN $\gamma$
Behandlung: 0,2 mg/m$^2$ (entsprechend 0,06 mg, ca. 10$^6$ I.U.) i.m. für sechs Tage. Danach Reduzierung auf die halbe Dosis für weitere 4 Tage wegen auftretender Nebenwirkungen (Fieber, Tachykardie).

Ergebnis: Rapides, völliges Abheilen der Zungenläsion innerhalb von 20 bis 24 Tagen nach Therapiebeginn, verbunden mit deutlicher Verbesserung des klinischen Zustandes des Kindes. Parallel dazu erfolgte eine Verbesserung des Immunstatus durch Rückgang der Lymphozyten-Subpopulation CD-19 von 32%auf 7% (bzw. 16%), welche durch die entsprechenden und bekannten monoklonalen Antikörper über die sog. CD (Cluster Defined)-Antigene quantifiziert wurde. Die erfolgte Zunahme der OKT-9 (Transferrin-Rezeptor) positiven Zellpopulationen von 1% auf 6% kann gleichermaßen als Besserung der Immunlage gedeutet werden.

**Patentansprüche**

1. Verwendung von Interferon-gamma (IFN$\gamma$) zur Herstellung eines Arzneimittels zur Verhütung und Behandlung von durch primäre Immundefekte verursachte Krankheiten.

2. Verwendung von IFNγ nach Anspruch 1 zur Verhütung und Behandlung von durch humorale primäre Immundefekte verursachte Krankheiten.

3. Verwendung von IFNγ nach Anspruch 1 zur Verhütung und Behandlung von durch zelluläre primäre Immundefekte verursachte Krankheiten.

4. Verwendung von IFNγ nach Anspruch 1 zur Verhütung und Behandlung von durch kombinierte primäre Immundefekte (SCID) verursachte Krankheiten.

5. Verwendung von IFNγ nach Anspruch 1 zur Verhütung und Behandlung von durch das Leucocyte Function Antigen (LFA 1) Defizienz Syndrom verursachte Krankheiten.

6. Verwendung von IFNγ nach einem der Ansprüche 1 bis 5, dadurch gekennzeichnet, daß das IFNγ natürliches, synthetisches, semisynthetisches oder über DNA-Rekombination hergestelltes IFNγ ist.

7. Verwendung von IFNγ nach einem der Ansprüche 1 bis 6, dadurch gekennzeichnet, daß das IFNγ ein gegenüber dem genuinem IFNγ verändertes Derivat mit IFNγ-Aktivität ist.

8. Verwendung von IFNγ nach einem der Ansprüche 1 bis 7, dadurch gekennzeichnet, daß das IFNγ in Kombination mit mindestens einem antibakteriellen und/oder antiviralen und/oder antimykotischen Wirkstoff vorliegt.

## Claims

1. Use of interferon-gamma (IFNγ) in the preparation of a pharmaceutical composition for preventing and treating diseases caused by primary immunodeficiencies.

2. Use of IFNγ according to claim 1 in the prevention and treatment of diseases caused by humoral primary immunodeficiencies.

3. Use of IFNγ according to claim 1 in the prevention and treatment of diseases caused by cellular primary immunodeficiencies.

4. Use of IFNγ according to claim 1 in the prevention and treatment of diseases caused by combined primary immunodeficiencies (SCID).

5. Use of IFNγ according to claim 1 in the prevention and treatment of diseases caused by Leucocyte Function Antigen (LFA1) deficiency syndrome.

6. Use of IFNγ according to one of claims 1 to 5, characterised in that the IFNγ is natural, synthetic or semi synthetic IFNγ or IFNγ produced by DNA recombination.

7. Use of IFNγ according to one of claims 1 to 6, characterised in that the IFNγ is a derivative with IFNγ activity which has been modified from genuine IFNγ

8. Use of IFNγ according to one of claims 1 to 7, characterised in that the IFNγ is present in conjunction with at least one antibacterial and/or antiviral and/or antimycotic active substance.

## Revendications

1. Utilisation de l'interféron gamma (IFNγ) pour la préparation d'un médicament pour la prévention et le traitement de maladies provoquées par des déficiences immunitaires primaires.

2. Utilisation d'IFNγ selon la revendication 1 pour la prévention et le traitement de maladies provoquées par des déficiences immunitaires primaires humorales.

3. Utilisation d'IFNγ selon la revendication 1 pour la prévention et le traitement de maladies provoquées par des déficiences immunitaires primaires cellulaires.

4. Utilisation d'IFNγ selon la revendication 1 pour la prévention et le traitement de maladies provoquées par des déficiences immunitaires primaires combinées (SCID).

5. Utilisation d'IFNγ selon la revendication 1 pour la prévention et le traitement de maladies provoquées par le syndrome de déficience en leucocyte function antigen (LFA 1).

6. Utilisation d'IFNγ selon l'une des revendications 1 à 5, caractérisée en ce que l'IFNγ est un IFNγ naturel, synthétique, semisynthétique ou préparé par recombinaison d'ADN.

7. Utilisation d'IFNγ selon l'une des revendications 1 à 6, caractérisée en ce que l'IFNγ est un dérivé modifié par rapport à l'IFNγ authentique, qui présente une activité IFNγ.

8. Utilisation d'IFNγ selon l'une des revendications 1 à 7, caractérisée en ce que l'IFNγ est présent en combinaison avec au moins un principe actif antibactérien et/ou antiviral et/ou antimycotique.